Europäisches Patentamt

(19) European Patent Office

Office européen des brevets

(11) Publication number: **0 002 541**
A1

## (12) EUROPEAN PATENT APPLICATION

(21) Application number: 78101761.1

(22) Date of filing: 19.12.78

(51) Int. Cl.²: **G 01 S 7/62**
**H 04 N 3/34, A 61 B 10/00**

(30) Priority: 19.12.77 US 861667

(43) Date of publication of application:
27.06.79 Bulletin 79/13

(84) Designated contracting states:
DE FR GB NL SE

(71) Applicant: PICKER CORPORATION
595 Miner Road
Cleveland, Ohio 44143(US)

(72) Inventor: Adams, Darwin P.
680 Tanner Marsh Road
Guilford Connecticut 06472(US)

(74) Representative: Baillie, Iain Cameron et al,
c/o Ladas, Parry, von Gehr. Goldsmith & Deschamps
Blumenstrasse 48
D-8000 München 2(DE)

(54) Apparatus and method for producing an image of internal structure of a subject.

(57) Apparatus and method producing an image of internal structure of a subject (P), suitable for use in an electronically scanned linear array real time ultrasonic system. The apparatus includes an ultrasonic transducer (T) for propagating incident ultrasonic energy into a subject (P) and for producing electrical signals representing ultrasonic echoes from within the subject which are produced in response to the incident energy. Imaging circuitry (I) processes the electrical signals to actuate a CRT display apparatus (D) for generating a raster formatted image of internal subject structure, with an electron beam on a tube face. A random noise generator (N) applies a random noise modulation signal to deflection plates of the CRT display for rapidly deflecting the electron beam of the CRT to wobble transversely with respect to the raster line (L) during image generation.

Fig. I

EP 0 002 541 A1

- 1 -

APPARATUS AND METHOD FOR PRODUCING AN IMAGE
OF INTERNAL STRUCTURE OF A SUBJECT

This invention relates to ultrasonic imaging techniques, and particularly to means for enhancing the quality of ultrasonically derived images by means of improved ultrasonic image display apparatus and method.

This invention is applicable to the field of electronically scanned real time linear array ultrasonic imaging systems. Such a system includes (1) a transducer assembly having a linear array of elements for directing ultrasonic energy into a subject and producing electrical signals in response to ultrasonic echoes thus caused, (2) imaging electronics including circuitry for processing the electrical signals from the transducer and (3) a CRT display apparatus for generating an ultrasonically derived visual image of internal subject structure in response to the processed electrical signals.

Combinations of the transducer elements are electrically actuated by the imaging electronics to direct ultrasonic energy into the subject. The imaging electronics sequentially actuates the various combinations of transducer elements in accordance with a predetermined pattern. The transducer element combinations are actuated such that the transducer assembly as a whole produces a particular sequence of resultant ultrasonic beams, these resultant beams emanating effectively from a succession of locations relative to the transducer assembly.

The CRT display produces an image in a raster format. In producing the raster image, the imaging electronics causes the display apparatus to produce each respective raster image line in a predetermined relation with the production of one of the sequence of resultant ultrasonic beams. The relation is determined such that the spatial region of the subject represented by each raster line corresponds to the actual region of the subject from which the echo causing the production of that raster line emanates.

While such systems have proven useful, it has for some time been recognized that specific improvements in image quality are desirable. One problem with the images produced by such systems is that often the raster lines of the displayed image are visually distinguishable one from another, impairing somewhat the resolution and subjective realism of the image. Another area in which improvement has been considered desirable is that of filling the blank spaces between raster lines with estimated or interpolated brightness, a feature not attainable satisfactorily in prior systems.

This "filling in" achieves a gradual variation of the light intensity between adjacent raster lines, and the consequent reduction of the appearance of relatively dark lines in the spaces between adjacent raster lines. Clearly, a high quality interpolation between the lines is preferable. The achievement of first order interpolation, for example, causes the various image raster lines to appear to blend with one another in a pleasing fashion.

Numerous proposals have been made for filling in between the lines, thereby making the image more pleasing and informative. It has been found difficult, however, to simultaneously (1) diffuse or blend the raster lines to eliminate distracting abrupt variances in light levels between the lines, and (2) maintain good resolution and (3) achieve high quality interpolation between the lines.

The previous proposals include superimposing on the display deflection plates a sinusoidal periodic signal in addition to processed electrical and raster producing signals, for deflecting the electron beam of the display transverse to the raster lines, in order to blur them. It has also been proposed to selectively defocus the electron beam itself, to spread the beam. Both of these proposals have proved lacking.

It has been found that, when a sinusoidal periodic signal is applied to deflect the electron beam transverse to the raster lines, a poor quality interpolation results. The interpolation between the lines becomes similar to that of a "zero order," rather than a first order, or linear, interpolation. If a sine wave is superimposed on the plates the beam dwells longer near the extremities of its excursion about the raster line than near the center of the line. The result is that the line brightness is concentrated away from the line center, rather than increasing gradually toward its center from a darker region between the lines. Such phenomena result in abrupt changes in the light levels between adjacent lines, rather than a desirable and pleasing variation. This increases the noticeability of the raster format, and makes more difficult the production of a realistic image.

Another disadvantage in the use of periodic functions is that good quality periodic function generators must avoid interference phenomena such as Moire' patterns and may require additional circuitry to maintain proper adjustment, gain level and synchronization. This extra circuitry adds cost and can be associated with low reliability.

Defocusing or astigmating the electron beam, while serving to blur the raster lines, has been considered disadvantageous because it is very difficult to defocus the beam and maintain Gaussian beam distribution. Also, unless special CRT design is used, when the electron beam is astigmated or defocused, the beam diameter increases, and it becomes more difficult to develop an image illustrating fine detail.

The foregoing deficiencies of the described prior art systems are overcome by the apparatus and method of this invention.

The ultrasonic apparatus embodying this invention produces an image of internal structure of a subject by the use of ultrasonic energy. The apparatus includes an ultrasonic transducer having means for directing ultrasonic energy into the subject and for producing representative electrical signals in response to echoes produced by the ultrasonic energy within the subject. The apparatus also includes circuitry for processing the electrical signals and a display apparatus responsive to the processed electrical signals for producing the image.

The display apparatus includes a display medium, apparatus for producing an energy beam and means for deflecting the electron beam about the medium in a pattern of lines making up a raster.

The apparatus further includes a random noise generator coupled to the display apparatus for additionally deflecting the energy beam in response to the random noise signal. The additional deflection is transverse to the lines of the raster pattern.

The use of random noise to deflect the electron beam blurs the raster lines in a desirable manner, blending them together, without degrading the resolution of the image. In fact, this manner of diffusing the raster lines actually improves apparent image quality, resulting in a smoother, more pleasing picture than without the random noise superimposition.

The improved picture results from a high quality interpolation caused by the use of random noise having a substantially Gaussian amplitude distribution, in accordance with a specific aspect of the invention. Additionally, the image is generally regarded as subjectively more pleasing than without the random noise usage, in that the raster lines tend to blend naturally with one another.

With the use of random noise having a Gaussian distribution, the light levels between adjacent raster lines having differing brightness tend to vary in a linear fashion, rather than abruptly, such as occurred with systems employing sine wave modulation.

Furthermore, interference phenomena which can occur with periodic functions are avoided when random noise is used. This results in less critical adjustment of gain and synchronization and thereby reduces system cost.

According to a more specific feature of the invention, the random noise generator includes a noise doide and means for applying an electrical potential across the noise diode. Since the noise generator produces its noise relatively continuously without the aid of adjustment, there is no concern about maintaining the uniformity of the superimposed noise modulation signal.

In accordance with another specific aspect of the invention, the amplitude of the random noise can be adjusted. Such adjustability enables varying the degree of diffusion of the raster lines to accommodate for changes in the distances between adjacent raster lines used to constitute an image.

In accordance with another specific feature of this invention, the random noise signal is applied to the display apparatus by means of capacitive coupling to the deflection plates of the cathode ray display device.

This invention will be understood in more detail with reference to the following claims and detailed description.

In the drawings:

FIGURE 1 is partially graphic, partially block diagram illustrating an ultrasonic imaging system incorporating the present invention.

FIGURE 2 is a block diagram illustrating a portion of the system of FIGURE 1.

FIGURES 3 and 4 are schematic drawings illustrating in detailed form portions of the system shown in FIGURE 2.

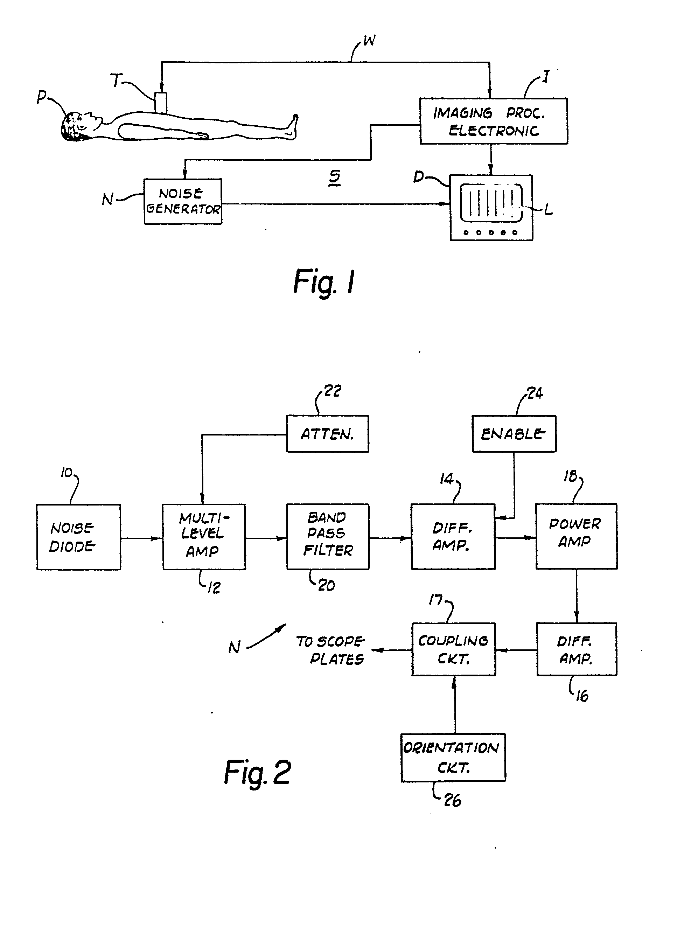

A system S incorporating the present invention is illustrated in FIGURE 1. The system S includes a transducer T connected to imaging electronics I by way of a set of leads W, and a display apparatus D connected to an output
of the imaging electronics I. The transducer T is actuated
by the electronics I to direct ultrasonic energy into a
subject, such as a patient P. The ultrasonic energy causes
the occurrence of echoes within the patient P at interfaces
between his various body tissues. Some of the echoes return
to the transducer T, which produces electrical signals over
the leads W to the imaging electronics I. The electronics
I processes the electrical signals and directs them to the
display apparatus D which, in response, produces a visual
image, derived from the processed electrical signals, which
represents internal structure of the body of the patient P.
The electronics I can preferably also produce signals causing the display to produce a reference marker in the image.

The display apparatus D produces the image of
internal subject structure in accordance with a raster format. The raster consists of a series of lines L whose
brightness varies with location to form a pattern representing the internal subject structure.

The transducer T includes a linear array of individual transducer elements (not shown). The imaging electronics, in actuating the transducer, stimulates a predetermined sequence of element combinations such that the transducer T produces a sequence of resultant ultrasonic beams
effectively emanating from a succession of locations relative to the transducer T.

The imaging electronics coordinates the production
of the raster lines and ultrasonic beams so that the subject
region represented by each raster line corresponds to the
subject region penetrated by the beam from which the visual
information of the raster line is derived.

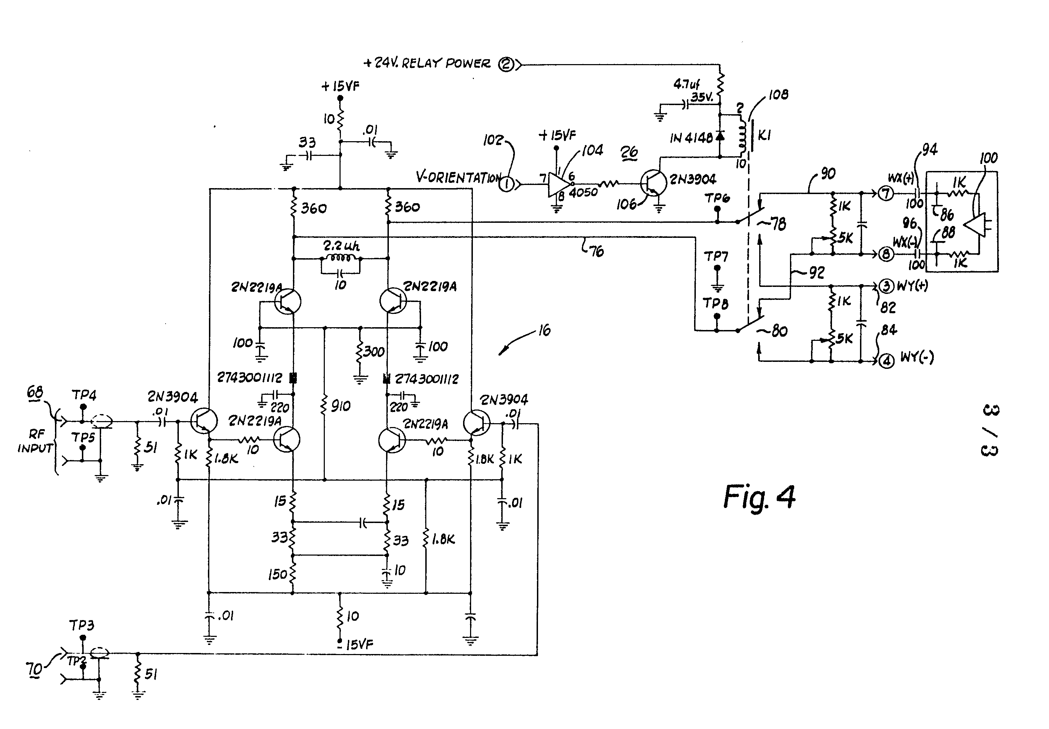

The display D is suitably embodied by a cathode
ray tube oscilloscope. The oscilloscope includes a common
cathode ray tube with a face constituting a display medium
within an envelope, and an electron gun. The scope also
includes horizontal and vertical plates for controllably
deflecting electron beam from the gun about the tube face,
and internal oscillator circuitry (FIGURE 4) coupled to the plates for deflecting the beam to form a raster. Suitable parameters for the raster include numbers of lines ranging from 40 to 240, with line sweep times between about 87 microseconds and 260 microseconds. The raster lines may be either vertical or horizontal.

The electron beam intensity is modulated by the processed electrical signals from the imaging electronics I to impart to the raster video information representing subject structure derived from the ultrasonic echoes.

The transducer T, the leads W, the imaging electronics I and the display apparatus D are suitably embodied by an electronically scanned linear array real time ultrasonic imaging apparatus manufactured by applicant and designated as Model LS-1000.

A noise generator N directs a random noise signal to the display apparatus D. The noise signal from the noise generator N is applied to the appropriate deflection plates of the display apparatus D to cause the electron beam of the cathode ray tube to rapidly oscillate or "wobble" transversely about each line, as the beam produces the line. If the lines L are vertical, the noise is applied to the horizontal plates; if the lines are horizontal, the noise is applied to the vertical plates.

The noise generation circuitry N is illustrated in block form in FIGURE 2. The noise generator N includes a noise diode 10, circuitry for amplifying and filtering the noise produced by the noise doide, and coupling circuitry for transmitting the noise to selected deflection plates of the cathode ray tube oscilloscope display apparatus D. The amplification circuitry for the noise signal includes a multilevel amplifier 12, differential amplifiers 14, 16 and a power amplifier 18. The filtering circuitry includes a band pass filter 20. The coupling circuitry is indicated at 17.

Other circuitry comprising the noise generator N includes an attenuator circuit 22, enabling circuitry 24 and orientation circuitry 26. The attenuator 22 adjusts the gain of the multilevel amplifier 12 to enable selected

- 6 -

variation of the amplitude of the noise produced by the noise generator N. The enabling circuitry 24 is used to turn on and off the differential amplifier 14 so that the application of the noise signal to the oscilloscope plates may be interrupted as desired, as when a reference index marker is generated in the image. The orientation circuitry 26 is connected to the coupling circuitry 17 for selectively applying the noise signal to the desired ones of the horizontal and vertical deflection plates of the oscilloscope display D. This feature enables the deflection of the electron beam of the CRT transverse to either vertical or horizontal raster lines.

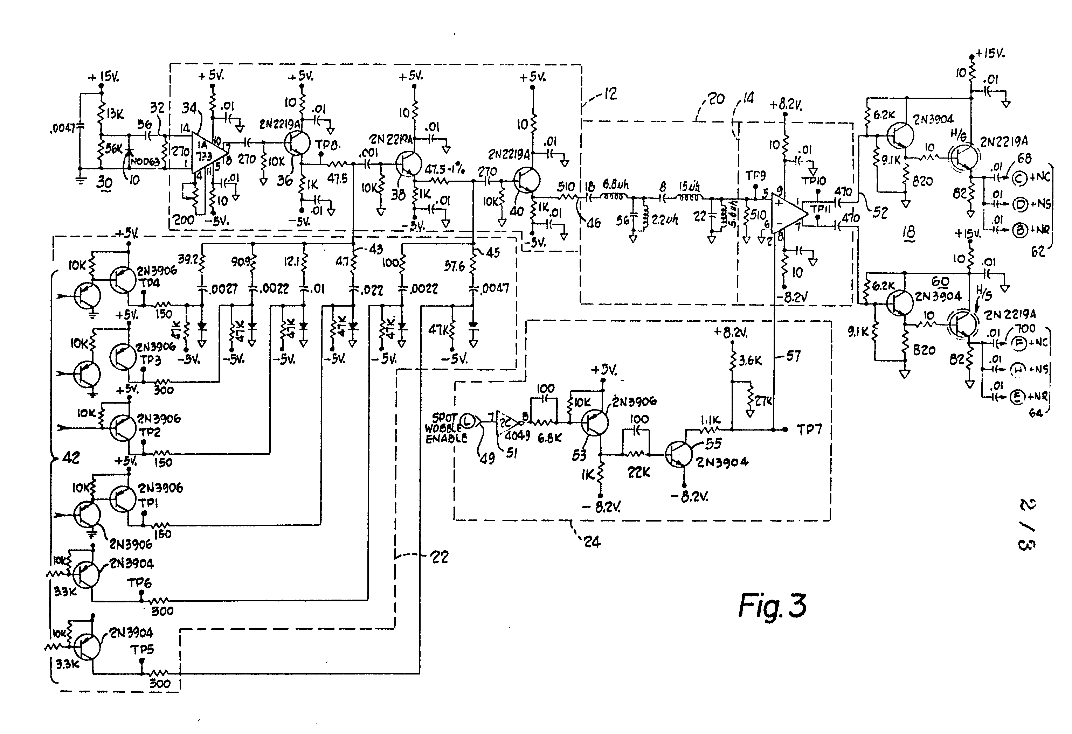

The features of the noise generator N are illustrated schematically in FIGURES 3 and 4. Referring first to FIGURE 3, the noise diode 10 is preferably indicated as an NOD 63 diode and is shown in connection with its associated supply circuitry 30. The signal from the noise diode 10 is a random noise signal having a substantially Gaussian amplitude distribution, and a frequency distribution extending from about 1 MHz through 20 MHz and which appears on a lead 32.

The multilevel amplifier 12 is indicated within the correspondingly numbered dotted box in FIGURE 3, and its principal elements include an operational amplifier 34 and transistors 36, 38, 40. The multilevel amplifier 12 amplifies the noise signal in an amount which is determined by which combination of a set 42 of attenuator input leads bears a signal. The attenuator circuitry 22, shown within the correspondingly numbered dotted box of FIGURE 3, includes amplification circuitry and attenuator outputs 43, 45, 47 illustrated in FIGURE 3 as coupled to vary the gain of the multilevel amplifier 12. The gain of the amplifier 12 can be varied in known fashion in steps from about Odb to 22db.

Preferably, the raster image produced by the display apparatus D has between 40 and 240 lines. The fewer the lines in the raster used to produce the image, the farther apart are the individual adjacent lines. When the individual adjacent lines are relatively far apart, it is desirable to raise the gain on the multilevel amplifier 12 to produce a greater deflection of the electron beam and a greater spreading or blending of the raster lines. When a raster having a greater number of lines is used, the individual lines are closer together, and a lower amplification of the noise signal is desirable. This adjustment is obtained by varying combination of signals on the attenuator inputs 42 of the attenuator circuitry 22.

The band pass filter 20 is illustrated within the correspondingly numbered dotted box in FIGURE 3 and has a pass band of about 10 to 20 MHz. The filter 20 passes noise signals from a lead 46 to an output point 48.

The differential amplifier 14, having as its primary component an operational amplifier 50, is illustrated in FIGURE 3 as having complementary outputs appearing on leads 52, 54. Each of the complementary outputs is transmitted through respective power amplifier stages 58, 60, to appear respectively at sets of output terminals 62, 64.

The enablement circuitry 24 is also shown in FIGURE 3. This circuitry includes an input 49, and inverter 51, transistors 53, 55 and an output lead 57. When it is desired to temporarily interrupt operation of the noise generator N, as during the production of an image reference marker, a signal is applied, e.g., from the circuitry I, at the input 49 producing an output on the lead 57, disabling the differential amplifier 14. When the signal at input 49 is removed, the amplifier 14 and the generator N resume normal operation. Interruption of generator N operation during marker production is desirable to assure the clearest possible definition of the marker.

In FIGURE 3, each of the sets 62, 64 of output terminals include three terminals for each of the complementary noise signals. The subsequent discussion will deal with the outputs from only one of the terminals of each of the sets 62, 64, it being understood that the remaining terminals of the sets 62, 64 can be coupled to other display apparatus (not shown) for improving the picture not only on the display apparatus D, but also on additional display units.

Referring now to FIGURE 4, the circuitry illustrated there shows the transmission of the complementary noise signals appearing on terminals 68,70, illustrated in FIGURE 3. The noise signals on the leads 68,70 are transmitted over corresponding inputs to the differential amplifier generally indicated as 16. The noise output from the differential amplifier 16 appears on the leads 74, 76, and is transmitted to a set of relays 78, 80, which together constitute a portion of the coupling circuitry 17.

The positions of the relays 78, 80 determines whether the noise signal is transmitted to the horizontal or vertical deflection plates of the cathode ray oscilloscope. If the relays 78, 80 are in their lower positions, the noise signals are transmitted to the vertical deflection plates (not shown) by way of a pair of leads 82, 84. If the relays 78, 80 are in their upper positions, the noise signal is transmitted to the horizontal deflection plates 86, 88 of the oscilloscope over a pair of leads 90, 92.

The noise signal on the leads 90, 92 is reactively coupled to the plates 86, 88 by way of a pair of capacitors 94, 96. The capacitors 94, 96 serve to transmit substantially only the AC component of the noise and to eliminate any undesirable displacement of the individual raster lines of the display image which might occur if the noise component included a resultant DC value.

A portion of the cathode ray oscilloscope of the display D, including an internal oscilloscope amplifier 100, (for producing the raster) is shown in FIGURE 4 as being connected in parallel with the noise signal to the deflection plates 86, 88.

The orientation circuitry 26 is illustrated in FIGURE 4. The orientation circuitry includes an orientation input 102, an inverter 104 and a transistor 106, along with a solenoid 108 and associated circuitry. When a signal is applied to the terminal 102, it is subsequently applied to actuate the solenoid 108, which actuates the relays 78, 80

- 11 -

to move to their upward position and to apply the noise signal to the horizontal deflection plates 86, 88. When the signal is removed from the terminal 102, the solenoid 108 actuates the relays 78, 80 to move to their lower positions which causes the noise signal to be directed onto the leads 82, 84 and, as explained above, subsequently to the vertical deflection plates of the cathode ray oscilloscope.

CLAIMS

1. Apparatus for producing an image of internal structure of a subject, said apparatus being responsive to image position indicating signals produced by an ultrasonic imaging system, characterized in that said apparatus comprises: apparatus for producing a raster display including a pattern of lines; circuitry operable upon the raster producing apparatus to cause the production of an image in response to the position indicating signals; and circuitry for applying random noise to the raster producing apparatus diffusing the raster lines.

2. The apparatus of claim 1, characterized in that the raster producing apparatus includes: a display medium; means for producing and deflecting an incident energy beam about the medium; and means for modulating the energy beam intensity with the position indicating signals, and the noise applying circuitry comprises means for applying random noise to the beam deflecting means for deflecting the beam transverse to the raster lines.

3. Apparatus for producing an image of internal structure of a subject by the use of ultrasonic energy, said system being characterized by: transducer apparatus for producing electrical signals from ultrasonic echoes, said electrical signals representing characteristics of the subject structure; circuitry for processing the electrical signals, and display apparatus comprising: apparatus for producing a raster display including a pattern of lines; circuitry for modulating the raster producing apparatus as a function of the processed electrical signals to cause the raster producing apparatus to generate an image of the subject structure, and circuitry for applying random noise to the raster producing apparatus for diffusing the raster lines.

4.   Apparatus for producing an image of internal structure of a subject by the use of ultrasonic energy, said system being characterized by:  an ultrasonic transducer including means for directing ultrasonic energy into the subject, and means for producing representative electrical signals in response to echoes produced by the ultrasonic energy; circuitry for processing the electrical signals; display apparatus responsive to processed electrical signals for producing the image, including a display medium; apparatus for producing an energy beam directed toward the display medium and modulated by the processed electrical signals; and, circuitry for deflecting the energy beam about the display medium in a pattern of lines comprising a raster; and a random noise generator coupled to the display apparatus for additionally deflecting with random noise the energy beam transverse to the lines of said raster pattern.

5.   The apparatus of claim 4, characterized in that said noise generator includes a noise diode.

6.   The apparatus of claim 4, characterized in that said display apparatus includes a cathode ray oscilloscope having a cathode ray tube, an electron gun for producing an electron beam, a set of deflection plates associated with the cathode ray tube, and an oscilloscope generator coupled to the deflection plates for applying deflecting signals to the deflection plates; and further characterized by coupling circuitry between the noise generator and the deflection plates for applying said random noise to the deflection plates in parallel with the deflection signals.

7.   The apparatus of claim 4, characterized in that said random noise generator comprises means for producing a random noise signal having an approximately Gaussian amplitude distribution, and a frequency distribution range of approximately 10 to 20 megahertz, said noise generator being capacitatively coupled to said deflection plates, and including circuitry for causing the display apparatus to produce an index on said display medium, and circuitry for suspending the application of said noise signal during the production of said index.

8. The apparatus of claim 4, characterized by means for adjusting the amplitude of the random noise.

9. The apparatus of claim 4, characterized in that the beam deflecting circuitry includes vertical and horizontal deflection plates and, the apparatus further comprises circuitry for directing the random noise signal separately a selected one of the horizontal and vertical deflection plates.

10. The apparatus of claim 4, characterized by means for interrupting operation of the noise generator in response to another electrical signal.

11. Apparatus for producing a visual image of internal structure of a subject by the use of ultrasonic energy, characterized by an ultrasonic transducer comprising piezoelectric means for producing ultrasonic energy for direction into the subject and for responding to echoes produced by the ultrasonic energy to generate representative electrical signals; and power circuitry for actuating the piezoelectric means to produce the ultrasonic energy; processing circuitry connected to the transducer for receiving and processing the electrical signals; a cathode ray oscilloscope for producing the visual image in response to the processed signals, said oscilloscope comprising: a cathode ray tube having an electron gun and a screen; a set of deflection plates associated with the cathode ray tube; circuitry for actuating the electron gun to direct an electron beam onto the screen; an oscilloscope generator coupled to the deflection plates for applying deflection signals to the deflection plates for deflecting the electron beam in a raster comprising a pattern of lines; a random noise generator including a noise diode, and means for applying an electrical potential to the noise diode to generate a random noise signal; and reactive coupling circuitry between the noise generator and the deflection plates for applying the random noise signal to the deflection plates in parallel with said deflecting signals for additionally deflecting the electron beam transverse to the lines of said pattern.

12. A method of producing a visual image of internal structure of a subject by the use of ultrasonic energy, characterized by the steps of: directing ultrasonic energy into the subject; detecting ultrasonic energy echoes; producing representative electrical signals in response to the echoes; generating an energy beam; producing a raster display by deflecting the energy beam about a display medium in a pattern of lines; modulating the intensity of the energy beam in response to the electrical signals; producing a random noise signal; and, additionally deflecting the energy beam transverse to the lines of said pattern in response to the random noise signal.

13. The method of claim 12, characterized in that said random noise signal producing step comprises applying an electrical potential across a noise diode.

14. The method of claim 12, characterized in that said energy beam producing step comprises actuating the electron gun of a CRT oscilloscope to produce an electron beam; said deflecting step comprises actuating an oscilloscope generator to produce electron beam deflecting signals and to apply the deflecting signals to deflecting plates, and said additional deflecting step comprises applying the noise signal to the deflecting plates in parallel with the deflecting signals.

15. The method of claim 14, characterized by applying the random noise signal to the deflecting plates by means of reactive coupling, said random noise signal having an amplitude distribution which is approximately Gaussian; and a frequency distribution range of approximately 10 to 20 megahertz, and by producing a reference index on said display medium by the use of said energy beam, and suspending said additional deflecting step during the production of said reference index.

Fig. 1

Fig. 2

Fig. 3

Fig. 4

European Patent Office

**EUROPEAN SEARCH REPORT**

Application number

EP 78 10 1761

| Category | DOCUMENTS CONSIDERED TO BE RELEVANT | | CLASSIFICATION OF THE APPLICATION (Int. Cl.²) |
|---|---|---|---|
| | Citation of document with indication, where appropriate, of relevant passages | Relevant to claim | |
| | JEE JOURNAL OF ELECTRONIC EN-GINEERING, no. 132, december 1977 (TOKYO, JP) ITO et al.: "A real-time ultrasonic diagnostic system for dynamic and still images: Wireless Echo-vision", (published Dec., 3, 1977) pages 20-26 <br><br> * Page 20, left-hand column; abstract; page 21; figures 1-3; left-hand column, lines 13-22; page 11, figure 4; left-hand column, lines 14-24, right-hand column, lines 11-19; page 23; figure 5; page 24; photo 3; left-hand column, lines 8-26 * <br><br> -- <br><br> US - A - 2 904 629 (SCHERBATSKOY) <br><br> * Column 1, lines 15-18; column 1, line 45 - column 2, line 25; column 4, lines 3-6, 31-36 and 61-70; column 5, lines 4-50; column 6, claim 2; figure * <br><br> -- | 1-4,6, 9,11,12 14 <br><br><br><br><br><br><br><br><br><br><br><br><br><br><br><br> 1-4,6-9,11, 12,14 | G 01 S 7/62 <br> H 04 N 3/34 <br> A 61 B 10/00 <br><br><br><br><br><br> **TECHNICAL FIELDS SEARCHED (Int.Cl.²)** <br><br> G 01 S 7/56 <br> 7/62 <br> 9/66 <br> G 10 K 11/10 <br> H 04 N 3/34 <br> A 61 B 10/00 |
| A | US - A - 3 864 660 (RANALLI et al.) <br><br> * The complete document * <br><br> ---- | 1,11, 12 | **CATEGORY OF CITED DOCUMENTS** <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons <br><br> &: member of the same patent family, corresponding document |

The present search report has been drawn up for all claims

| Place of search | Date of completion of the search | Examiner |
|---|---|---|
| The Hague | 14-03-1979 | OLDROYD |

EPO Form 1503.1  06.78